# EUROPEAN PATENT SPECIFICATION

(11) **EP 2 137 176 B1**
(45) Date of publication and mention of the grant of the patent: **26.01.2011**
(21) Application number: 08736323.0
(22) Date of filing: 17.04.2008
(51) Int. Cl.: C07D 317/36

(54) **PROCESS FOR PREPARING AN 1,2-ALKYLENE CARBONATE**
VERFAHREN ZUR HERSTELLUNG EINES 1,2-ALKYLENCARBONATS
PROCÉDÉ DE PRÉPARATION D'UN CARBONATE DE 1,2-ALKYLÈNE

(30) Priority: 23.04.2007 EP 07106712
(43) Date of publication of application: 30.12.2009
(73) Proprietor: Shell Internationale Research Maatschappij B.V., 2596 HR The Hague (NL)
(72) Inventor: NISBET, Timothy Michael, NL-1031 CM Amsterdam (NL); VAPORCIYAN, Garo Garbis, Houston, TX 77079 (US)
(74) Representative: Matthezing, Robert Maarten
(86) International application number: PCT/EP2008/054659
(87) International publication number: WO 2008/128956

(56) References cited:
- WO-A-00/20407

## Description

The present invention relates to a process for preparing an 1,2-alkylene carbonate.

Processes for the production of 1,2-alkylene carbonates are known. WO-A 2005/003113 discloses a process in which carbon dioxide is contacted with an alkylene oxide in the presence of a suitable carbonation catalyst. The catalyst disclosed is a tetraalkylphosphonium compound. This specification discloses that the catalyst used has been recycled. The specification further discloses that the performance of the catalyst is very stable if the catalyst is recycled to the alkylene carbonate preparation in an alcohol, in particular in monopropylene glycol (1,2-propanediol). US-A 4,434,105 also discloses a process for the preparation of 1,2-alkylene carbonates. Various catalysts are disclosed. The document also describes that the catalyst after completion of the reaction may be reused.

In a continuous process the reaction product containing 1,2-alkylene carbonate and catalyst has to be subjected to a work-up treatment. Such work-up treatment generally includes one or more distillation steps to separate the 1,2-alkylene carbonate from the catalyst and other components. WO 00/20407 discloses such work-up treatment. In accordance with a first embodiment of WO 00/20407, the crude carbonation reactor effluent is treated as follows:
(a) subjecting the crude reactor effluent, in evaporator 20 which may be a wiped film evaporator or falling film tower, to low temperature evaporation to form a first evaporator overhead containing alkylene carbonate and an evaporator bottoms stream containing the catalyst, and recycling the evaporator bottoms stream to the reactor;
(b) removing the light components present in the first evaporator overhead to form a second evaporator overhead, and recycling the light components to the reactor;
(c) distilling, in distillation column 30, the second evaporator overhead to form a first distillation overhead stream and a first distillation bottoms stream containing alkylene carbonate, and recycling the first distillation overhead stream to the reactor;
(d) distilling, in distillation column 40, the first distillation bottoms stream to form a second distillation overhead stream containing alkylene carbonate and a second distillation bottoms stream, and recycling the second distillation bottoms stream to the reactor;
(e) distilling the second distillation overhead stream to form a third distillation overhead stream and a third distillation bottoms stream containing alkylene carbonate, and recycling the third distillation overhead stream to the reactor; and
(f) distilling the third distillation bottoms stream to form a fourth distillation overhead stream containing purified alkylene carbonate and a fourth distillation bottoms stream, and recycling the fourth distillation bottoms stream to the reactor.

The work-up treatment in accordance with the first embodiment of WO 00/20407 thus comprises a distillation sequence consisting of at least four distillation steps. Prior to the first distillation step, the carbonation catalyst and alkylene carbonate are separated as a bottoms stream and an overhead stream, respectively. Thereafter, the alkylene carbonate is separated in the first distillation step as a bottoms stream, in the second distillation step as an overhead stream, in the third distillation step as a bottoms stream, and finally in the fourth distillation step as an overhead stream. Therefore, WO 00/20407 teaches to recover purified alkylene carbonate as a distillation overhead stream, that is to say as a top product or distillate, rather than as a distillation bottoms stream.

An object of the present invention is to provide a process for preparing an 1,2-alkylene carbonate from carbon dioxide and an 1,2-alkylene oxide, using a carbonation catalyst, wherein the reactor effluent is treated in such way that the final 1,2-alkylene carbonate product contains no or substantially no contaminants and that only a limited number of separation steps is required to arrive at such purified 1,2-alkylene carbonate.

It has been found that the above object can be achieved by a process for preparing an 1,2-alkylene carbonate comprising
(i) contacting carbon dioxide, an 1,2-alkylene oxide and a carbonation catalyst in a reactor to produce a crude reactor effluent containing carbon dioxide, light components, 1,2-alkylene carbonate and catalyst;
(ii) separating carbon dioxide and light components from the crude reactor effluent to form a bottoms stream containing 1,2-alkylene carbonate and catalyst;
(iii) distilling the bottoms stream formed in step (ii) to form a first distillation overhead stream containing 1,2-alkylene carbonate and a first distillation bottoms stream containing catalyst, and recycling at least part of the first distillation bottoms stream to the reactor; and
(iv) distilling the first distillation overhead stream to form a second distillation overhead stream and a second distillation bottoms stream containing 1,2-alkylene carbonate, and recycling at least part of the second distillation overhead stream to the reactor.

In accordance with the process of the present invention, which may be carried out continuously, only two distillations have to be performed after having removed the carbon dioxide and light components from the crude reactor effluent. As is demonstrated in the examples below, by distilling the 1,2-alkylene carbonate as an overhead stream in the first distillation and removing final 1,2-alkylene carbonate product as a bottoms stream in the second distillation, rather than distilling final 1,2-alkylene carbonate product as an overhead stream in the second distillation, no or substantially no contaminants are contained in said final product. As discussed above, in the distillation sequence of the first embodiment of WO 00/20407, 1,2-alkylene carbonate is removed as a bottoms stream in a first distillation from which the 1,2-alkylene carbonate is distilled as an overhead stream in a second distillation. Moreover, in accordance with said known embodiment, two additional distillations need to be performed in order to arrive at substantially pure 1,2-alkylene carbonate.

More especially, it has been found that with the present process any halide compound (e.g. bromohydrin) that may be formed during the reaction as by-product is removed from the final 1,2-alkylene carbonate product and cannot hinder any subsequent process step. Further it has been found that by recycling the halide by-products to the carbonation reactor together with the catalyst the catalytic behaviour of the system is improved.

Besides having no or substantially no contaminants in the final product, an additional advantage is that in the present process first the carbon dioxide and light components are separated, in step (ii), from the crude reactor effluent formed in step (i). This results in a relatively low vapour loading for the next separation step (iii) of the present process wherein the bottoms stream formed in step (ii) is distilled. As a consequence, the size of the distillation column wherein said step (iii) is carried out can be reduced considerably. The vapour loading for the separation unit of the first embodiment of WO 00/20407, however, is relatively high because the alkylene carbonate is overheaded together with the carbon dioxide and light components in a first separation step.

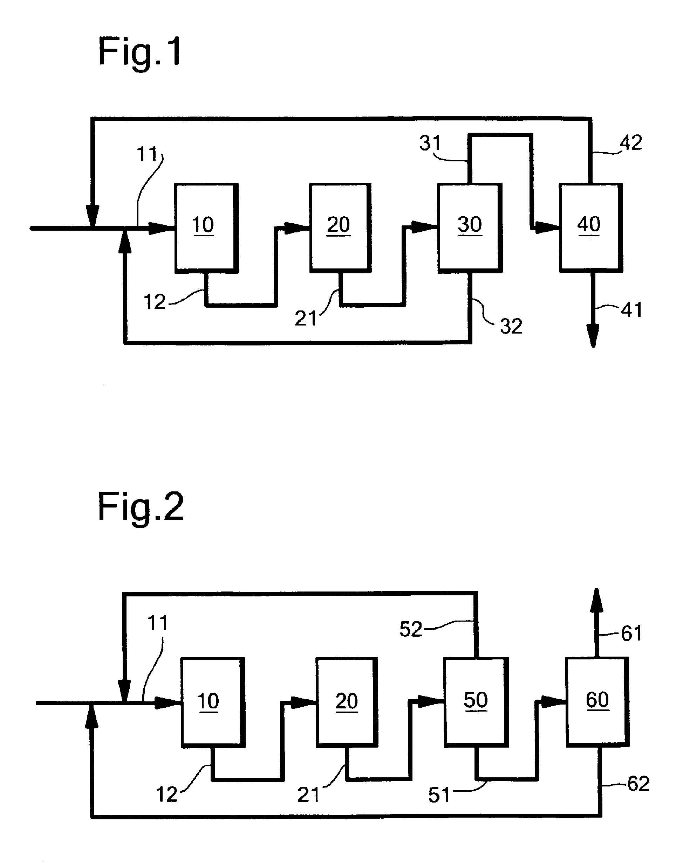

Fig. 1 schematically shows the process of the present invention. Each of steps (i) to (iv) of the present process is described below in more detail.

In step (i) of the present process, carbon dioxide, an 1,2-alkylene oxide and a carbonation catalyst are contacted in a reactor. As shown in Fig. 1, said three components are introduced into reactor 10 via line 11. Alternatively, they may be fed via different lines. For example, the 1,2-alkylene oxide may be fed via a separate line to the upper part of reactor 10; the carbon dioxide via a separate line to the middle part of said reactor; and the catalyst via a separate line to the lower part of said reactor.

The carbonation catalyst for use in the present invention generally will be a homogeneous catalyst, although a heterogeneous catalyst may also be used. A specific catalyst which is known to be suitable is a homogeneous phosphorus containing catalyst. The phosphorus will usually not be present in its elemental form in the catalyst. The carbonation catalyst may be a phosphonium compound. Such catalysts are known, e.g., from US-A 5,153,333, US-A 2,994,705, US-A 4,434,105, WO-A 99/57108, EP-A 776,890 and WO-A 2005/003113. Preferably, the catalyst is a phosphonium halide of formula R4PHal, in which Hal means halide and each R can be the same or different and can be selected from an alkyl, alkenyl, cyclic aliphatic or an aromatic group. Preferably, the carbonation catalyst comprises a tetraalkylphosphonium bromide. The group R suitably contains from 1 to 12 carbon atoms. Good results are obtained with R being a C₁₋₈ alkyl group. Most preferred are groups R being selected from methyl, ethyl, n-propyl, isopropyl, n-butyl, isobutyl, and t-butyl groups. Preferably, the halide ion is bromide or iodide. The most preferred phosphonium catalyst is tetra(n-butyl)-phosphonium bromide.

The catalyst may be recycled to the reactor as a solid, especially where the catalyst is a phosphonium catalyst. It is also possible to convert the catalyst to a melt and recycle the molten catalyst to the reactor. However, since the presence of a solvent shows a stabilising effect on the catalyst it is preferred to recycle the catalyst to the reactor in the presence of a solvent. The solvent can be a carbonyl-containing compound, especially aldehydes, as disclosed in WO-A 2005/051939. More preferably, the solvent is an alcohol. Many alcohols may be selected to increase the stability of the catalyst, especially a phosphonium catalyst. The alcohol may be monovalent, bivalent, or multivalent. The alcohol may comprise an aliphatic C₁₋₁₂ chain substituted by one or more hydroxyl groups. Aromatic alcohols or alkylaromatic alcohols may also be used, suitably having 6 to 12 carbon atoms. Polyalkylene glycols or the monoalkyl ethers thereof may also be used. Mixtures may also be used.

Preferably, the alcohols used are selected from the group consisting of C₁₋₆ mono-alkanols, C₂₋₆ alkane diols, C₃₋₆ alkane polyols, including glycerol, phenol, C₁₋₆ alkyl substituted phenols, C₆₋₁₂ cycloaliphatic alcohols and mixtures thereof. Very suitable are C₂₋₆ alkane polyols, in particular 1,2-ethanediol, 1,2-propanediol, sorbitol and mixtures thereof. The use of ethanediol or propanediol has a further advantage when the alkylene carbonate is converted to alkylene glycol (alkanediol), and the alkylene glycol is used as solvent for the catalyst. Sorbitol is providing excellent stability to the phosphonium catalyst. It may be advantageous to use a combination of 1,2-ethanediol or 1,2-propanediol and sorbitol.

Preferably, an 1,2-alkylene diol is used as a solvent for the carbonation catalyst. In such case, the first and second distillation overhead streams contain 1,2-alkylene diol.

Where an 1,2-alkylene diol is used as a solvent for the carbonation catalyst, the 1,2-alkylene diol is preferably monoethylene glycol and/or monopropylene glycol. Where such solvent is used, the 1,2-alkylene oxide may be ethylene oxide and the 1,2-alkylene diol may be monoethylene glycol, or the 1,2-alkylene oxide may be propylene oxide and the 1,2-alkylene diol may be monopropylene glycol.

Preferably, an 1,2-alkylene carbonate is used as a solvent for the carbonation catalyst. More preferably, the distillation in step (iii) is carried out in such way that part of 1,2-alkylene carbonate is contained in the first distillation bottoms stream, which 1,2-alkylene carbonate may then be used as solvent for the catalyst which is also contained in said stream. The 1,2-alkylene carbonate ensures that the used catalyst is in liquid form, which facilitates transportation, e.g., recycle.

Further, it has been found that the combination of alcohol and an 1,2-alkylene carbonate has a stabilising effect on the carbonation catalyst. The second distillation overhead stream suitably contains an alcohol, for example an 1,2-alkylene diol, in a case where such alcohol is used as a solvent for the carbonation catalyst. Therefore, if such alcohol is used, the first distillation bottoms stream and the second distillation overhead stream can suitably be combined such that a mixture of catalyst, alcohol and alkylene carbonate is recycled to the reactor.

In order to replenish any decomposed catalyst it may be effective to add make-up catalyst. The make-up catalyst can be added at any place in the present process where catalyst is present. Suitably any make-up catalyst is added to the process via direct addition to the reactor or via addition to the stream of catalyst that is to be recycled.

The amount of catalyst in the reactor may conveniently be expressed in mole catalyst per mole 1,2-alkylene oxide. Due to a lower amount of by-products, the subject process is suitably carried out in the presence of at least 0.0001 mole of the catalyst per mole 1,2-alkylene oxide. Preferably, the amount of catalyst present is such that it ranges from 0.0001 to 0.1 mole catalyst, more preferably from 0.001 to 0.05, and most preferably from 0.003 to 0.03 mole catalyst per mole 1,2-alkylene oxide.

The 1,2-alkylene oxide that is converted in the present process is suitably a C₂₋₄ alkylene oxide, preferably ethylene oxide and/or propylene oxide, or mixtures of such C₂₋₄ alkylene oxides.

The reaction of carbon dioxide with the 1,2-alkylene oxide is reversible. That means that the 1,2-alkylene carbonate formed may convert back into carbon dioxide and the 1,2-alkylene oxide. The molar ratio between carbon dioxide and 1,2-alkylene oxide may be as low as 0.5:1, more suitably from 0.75:1. In view of the reversibility of the reaction it is preferred to ensure at least a slight excess of carbon dioxide, such as 1.0:1 to 10:1, more preferably from 1.01:1 to 2:1, most preferably from 1.01:1 to 1.2:1. A suitable means to establish an excess of carbon dioxide is to conduct the reaction at an elevated carbon dioxide pressure and keeping the pressure constant by dosing carbon dioxide. The total pressure ranges suitably from 5 to 200 bar; the carbon dioxide partial pressure is preferably in the range from 5 to 70, more preferably from 7 to 50, and most preferably from 10 to 30 bar.

Besides providing for the desired surplus of carbon dioxide, operation at the above-mentioned increased pressures also permits to conduct the reaction essentially in the liquid phase, as 1,2-alkylene oxides such as ethylene oxide and propylene oxide will largely remain liquid under such process conditions.

The reaction temperature can be selected from a wide range. Suitably the temperature is selected from 30 to 300°C. The advantage of relatively high temperature is the increase in reaction rate. However, if the reaction temperature is too high, side reactions, i.a. the degradation of 1,2-alkylene carbonate to carbon dioxide and propionaldehyde or acetone, the undesired reaction of 1,2-alkylene oxide with any 1,2-alkanediol, if present, may occur, or the undesired decomposition of the catalyst may be accelerated. Therefore, the temperature is suitably selected from 100 to 220°C.

The skilled person will be able to adapt other reaction conditions as appropriate. The residence time of the 1,2-alkylene oxide and the carbon dioxide in the reactor can be selected without undue burden. The residence time can usually be varied between 5 min and 24 hours, preferably between 10 minutes and 10 hours. Conversion of 1,2-alkylene oxide is suitably at least 95%, more preferably at least 98%. Dependent on the temperature and pressure the residence time may be adapted. The catalyst concentration may also vary between wide ranges. Suitable concentrations include from 1 to 25 %wt, based on the total reaction mixture. Good results can be obtained with a catalyst concentration of 2 to 8 %wt, based on the total reaction mixture.

As to the relative amounts of 1,2-alkylene carbonate and alcohol, the latter only being used as a solvent for the catalyst, the skilled artisan can vary the ratio in broad ranges. Very good results have been obtained employing a weight ratio of 1,2-alkylene carbonate to alcohol of 1 to 100, in particular from 2 to 50, more preferably from 5 to 25. In view of the chance for the undesired reaction between the 1,2-alkylene oxide and an alcohol in the reactor the amount of alcohol is suitably kept at a relatively low level, such as from 1 to 25 %wt, based on the weight of 1,2-alkylene oxide, carbon dioxide, 1,2-alkylene carbonate and alcohol in the reactor. Preferably the amount of alcohol ranges from 5 to 20 %wt.

Where the catalyst is recycled to step (i) as part of a solution, it is advantageous if the content of the catalyst in such mixture to be recycled to step (i) is relatively high. That would mean that the yield of final 1,2-alkylene carbonate product is high whereas the costs for recycle are kept to a minimum. Therefore, the amount of catalyst in the mixture of catalyst and 1,2-alkylene carbonate ranges preferably from 1 to 90 %wt, based on the total mixture, more preferably from 5 to 75 %wt. Since it has been found that the stability of the catalyst is reduced slightly when the 1,2-alkylene carbonate to catalyst weight ratio is below 1 the amount of catalyst is most preferably from 10 to 40 %wt, the remainder comprising 1,2-alkylene carbonate and, optionally, alcohol.

In step (i) of the present process, only one reactor may be used. However, it is also feasible to carry out the reaction of step (i) in two or more reactors. In such cases it may be advantageous to provide for the optimal amount of excess carbon dioxide in the reactors by removing or adding carbon dioxide between the reactors. The reactors are suitably conducted under plug flow conditions. It is even more preferred to have a back-mix reactor, e.g. a Continuously Stirred Tank Reactor (CSTR), followed by a plug-flow reactor. Such a combination is known from e.g. US-A 4,314,945.

The carbon dioxide for use in the present process can be either pure carbon dioxide or carbon dioxide containing further compounds. Carbon dioxide which is especially suitable for use in the present invention, is carbon dioxide which has been separated off in any subsequent steps of the present process. The extent to which carbon dioxide is purified depends on the nature and the amounts of contaminants present in the carbon dioxide. Small amounts of water may be present in the carbon dioxide feed to the carbonation reactor. For example, water and the 1,2-alkylene oxide may react into 1,2-alkylene glycol. The 1,2-alkylene glycol produced in such way can easily be removed from the system, e.g. by bleeding or with 1,2-alkylene carbonate product, so as to maintain a desired level of 1,2-alkylene glycol if the 1,2-alkylene glycol is used as solvent for the catalyst.

In step (ii) of the present process, carbon dioxide and light components are separated from the crude reactor effluent to form a bottoms stream containing 1,2-alkylene carbonate and catalyst. As shown in Fig. 1, the crude reactor effluent leaves reactor 10 via line 12 and is then introduced into separator 20. In separator 20, the carbon dioxide and light components are separated. The bottoms stream formed in step (ii) is fed via line 21 to distillation column 30. Preferably, at least part of the separated light components and/or carbon dioxide is recycled to reactor 10 (not shown in Fig. 1). Separator 20 may consist of multiple, for example two, gas-liquid separators.

In accordance with the present description, light components are compounds, other than carbon dioxide, which have a boiling point which is lower than that of 1,2-alkylene glycols and 1,2-alkylene carbonates, more specifically 185°C or lower, and most specifically 180°C or lower. Examples of such light components in the crude effluent from the carbonation reactor may be unreacted 1,2-alkylene oxide and any light contaminants formed during the carbonation reaction, such as acetone, propionaldehyde, allyl alcohol and acetaldehyde.

Further, in accordance with the present description, an overhead stream comes from the top or from the upper part of a separation apparatus, such as a distillation column. Likewise, a bottoms stream comes from the bottom or from the lower part of a separation apparatus, such as a distillation column. Where a distillation column is used as a separation apparatus, this implies that an overhead stream may be discharged from either the highest tray or from a tray positioned below the highest tray, and that a bottoms stream may be discharged from either the lowest tray or from a tray positioned above the lowest tray.

In step (iii) of the present process, the bottoms stream formed in step (ii) is distilled to form a first distillation overhead stream and a first distillation bottoms stream. The first distillation overhead stream contains 1,2-alkylene carbonate. The first distillation bottoms stream contains catalyst. At least part of the first distillation bottoms stream is recycled to the reactor. If the present process is operated continuously, a bleed may optionally be taken from the recycle stream from step (iii) to step (i). Alternatively, no bleed is taken from said recycle stream and said stream is recycled entirely.

As shown in Fig. 1, in distillation column 30, the bottoms stream containing 1,2-alkylene carbonate and catalyst from separator 20 is distilled to form a first distillation overhead stream containing 1,2-alkylene carbonate which is fed via line 31 to distillation column 40. Further, a first distillation bottoms stream containing catalyst and possibly some 1,2-alkylene carbonate is formed, at least part of which is recycled via line 32 to reactor 10. Possibly, make-up catalyst may be added into line 32 or into any other suitable place in the process.

In a situation where an alcohol is used as a solvent for the catalyst and such alcohol has a lower boiling point than the 1,2-alkylene carbonate, as is the case when the alcohol used is 1,2-propanediol and the 1,2-alkylene carbonate is propylene carbonate or when the alcohol used is 1,2-ethanediol and the 1,2-alkylene carbonate is ethylene carbonate, the first distillation overhead stream contains said alcohol in addition to 1,2-alkylene carbonate. The first distillation overhead stream may also contain some light components which were formed during distillation in the distillation column, for example near the reboiler of said column.

As to the way the distillation may be performed in step (iii) in order to separate catalyst and possibly some 1,2-alkylene carbonate from 1,2-alkylene carbonate, any alcohol used as solvent for the catalyst and any light components, the skilled artisan can vary the temperature and number of trays without undue burden.

In step (iv) of the present process, the first distillation overhead stream is distilled to form a second distillation overhead stream and a second distillation bottoms stream. The second distillation bottoms stream contains 1,2-alkylene carbonate, i.e. the purified end product. At least part of the second distillation overhead stream is recycled to the reactor. If the present process is operated continuously, a bleed may optionally be taken from the recycle stream from step (iv) to step (i). Alternatively, no bleed is taken from said recycle stream and said stream is recycled entirely.

As shown in Fig. 1, in distillation column 40, the first distillation overhead stream containing 1,2-alkylene carbonate from distillation column 30 is distilled to form a second distillation bottoms stream containing final 1,2-alkylene carbonate product which is removed via line 41. Further, a second distillation overhead stream is formed, at least part of which is recycled via line 42 to reactor 10.

In a situation where an alcohol is used as a solvent for the catalyst and such alcohol has a lower boiling point than the 1,2-alkylene carbonate, the distillation in step (iv) should be carried out such that the second distillation overhead stream contains said alcohol and the final 1,2-alkylene carbonate product contains no or substantially no alcohol. The second distillation overhead stream may also contain some light components which were formed during distillation in step (iii) and/or step (iv) in the distillation column, for example near the reboiler of said column. Possibly, make-up alcohol may be added into line 42 or into any other suitable place in the process. The recycle stream from distillation column 40 may be sent directly to the reactor or may be mixed, in a separate vessel prior to entering the reactor, with the recycle stream containing catalyst and possibly some 1,2-alkylene carbonate from distillation column 30 (not shown in Fig. 1). Preferably, at least part of the first distillation bottoms stream and at least part of the second distillation overhead stream are mixed prior to recycling to step (i).

As to the way the distillation may be performed in step (iv) in order to separate 1,2-alkylene carbonate from any alcohol used as solvent for the catalyst and any light components, the skilled artisan can vary the temperature and number of trays without undue burden.

The 1,2-alkylene carbonate that is produced in the present process can suitably be used for the production of 1,2-alkanediol and dialkylcarbonate. Accordingly, the process of the present invention preferably comprises the following further steps:
(v) contacting at least part of the second distillation bottoms stream formed in step (iv) with an alkanol to obtain a reaction mixture containing an 1,2-alkylene diol and a dialkylcarbonate; and
(vi) recovering 1,2-alkylene diol and dialkylcarbonate.

The alkanol used in above transesterification step (v) is suitably a C₁₋₄ alcohol. Preferably, the alkanol is methanol, ethanol or isopropanol. Said step (v) may be performed in the presence of a heterogeneous transesterification catalyst.

The transesterification reaction in itself is known. In this context reference is made to US-A 4,691,041, disclosing a process for the manufacture of ethylene glycol and dimethyl carbonate by the transesterification reaction over a heterogeneous catalyst system, in particular an ion exchange resin with tertiary amine, quaternary ammonium, sulphonic acid and carboxylic acid functional groups, alkali and alkaline earth silicates impregnated into silica and ammonium exchanged zeolites. US-A 5,359,118 and US-A 5,231,212 disclose a continuous process for preparing dialkyl carbonates over a range of catalysts, including alkali metal compounds, in particular alkali metal hydroxides or alcoholates, such as sodium hydroxide or methanolate, thallium compounds, nitrogen-containing bases such as trialkyl amines, phosphines, stibines, arsenines, sulphur or selenium compounds and tin, titanium or zirconium salts. According to WO-A 2005/003113 the reaction of alkylene carbonate with an alkanol is conducted over heterogeneous catalysts, e.g. alumina.

The present invention will be further elucidated by means of the following examples.

### Comparative Example

Fig. 2 schematically shows the process carried out in this Comparative Example. In reactor 10 as shown in said Fig. 2, carbon dioxide, an 1,2-alkylene oxide and a carbonation catalyst were contacted. They were introduced into reactor 10 via line 11. The crude reactor effluent was sent from reactor 10 via line 12 to separator 20. In separator 20, carbon dioxide and light components were separated from the crude reactor effluent, to form a bottoms stream containing 1,2-alkylene carbonate and catalyst which was fed via line 21 to first distillation column 50. The separated light components and carbon dioxide were partially recycled to reactor 10 (not shown in Fig. 2).

The process as schematically shown in said Fig. 2 differs from that in Fig. 1, showing the process of the present invention, in the following two aspects.

First of all, in first distillation column 50 in Fig. 2, the bottoms stream containing 1,2-alkylene carbonate and catalyst from separator 20 was distilled to form a first distillation bottoms stream containing 1,2-alkylene carbonate and catalyst which was fed via line 51 to distillation column 60. Further, a first distillation overhead stream was formed, which was entirely recycled via line 52 to reactor 10.

Secondly, in distillation column 60 in Fig. 2, the first distillation bottoms stream containing 1,2-alkylene carbonate and catalyst from distillation column 50 was distilled to form a second distillation overhead stream containing final 1,2-alkylene carbonate product which was removed via line 61. Further, a second distillation bottoms stream was formed, which was entirely recycled via line 62 to reactor 10.

The 1,2-alkylene oxide used was propylene oxide which by reacting with the carbon dioxide resulted in propylene carbonate. The carbonation catalyst used was a homogeneous catalyst, namely tetra(n-butyl)phosphonium bromide, dissolved in a solution containing propylene carbonate, 20 wt.% of said catalyst and 20 wt.% of monopropylene glycol. Feed rates to reactor 10 were 114 g/h of propylene oxide, 60 normal litres/h of carbon dioxide (molar excess of carbon dioxide over propylene oxide) and 57 g/h of the catalyst solution. The pressure and temperature inside the carbonation reactor were 20 bar gauge and 150°C, respectively. Said reactor was a mechanically stirred autoclave having a volume of 1 litre.

The light components and carbon dioxide in the crude reactor effluent were removed in two gas-liquid separators in series (not shown in Fig. 2). The first one was operated at 24 bar gauge and 60°C and the second one at 4 bar gauge and 60°C. The light components and carbon dioxide from the first separator were recycled to the carbonation reactor. The light components and carbon dioxide from the second separator were vented to an incinerator header.

The crude bottoms stream containing propylene carbonate, monopropylene glycol and catalyst from the second gas-liquid separator was fed to the first of two distillation columns in series. The first distillation column was a glass Oldershaw column consisting of 17 trays, with the feed positioned 10 trays above the bottom reboiler. The temperature at the bottom of the first distillation column was 140°C and the pressure at the top was 0.030 bar absolute. The reflux ratio amounted to R/D = 1.6 (i.e. ratio of reflux flow to top product flow). The first distillation overhead stream contained monopropylene glycol and was entirely recycled, via a catalyst recycle vessel (not shown in Fig. 2), to the carbonation reactor. In addition, because at said temperature and pressure propylene carbonate forms an azeotrope with monopropylene glycol, the first distillation overhead stream also contained some propylene carbonate (about 15 wt.%). Even though the entire process was operated continuously, no bleed had to be taken from said recycle stream. The first distillation bottoms stream containing propylene carbonate and catalyst was fed to the second distillation column.

The second distillation column was a glass Oldershaw column consisting of 5 trays, with no trays below the feed. The temperature at the bottom of the first distillation column was 135°C and the pressure at the top was 0.030 bar absolute. The reflux ratio amounted to R/D = 0.3 (i.e. ratio of reflux flow to top product flow). The second distillation bottoms stream contained catalyst dissolved in propylene carbonate, the catalyst concentration in the bottoms product being about 25 wt.%, and was entirely recycled, via the above-mentioned catalyst recycle vessel where it was mixed with the recycle stream from the first distillation column, to the carbonation reactor. Even though the entire process was operated continuously, no bleed had to be taken from said recycle stream. The second distillation overhead stream contained the final propylene carbonate product.

### Example

Fig. 1 schematically shows the process carried out in this Example according to the present invention. The process carried out was identical to that carried out in the Comparative Example, with the exception that the first distillation column was the same as the second distillation column used in the Comparative Example, and the second distillation column was the same as the first distillation column used in the Comparative Example.

A further difference was that in this Example, the first distillation bottoms stream containing catalyst dissolved in propylene carbonate and the second distillation overhead stream containing monopropylene glycol (and some propylene carbonate) were entirely recycled, via the above-mentioned catalyst recycle vessel, to the carbonation reactor.

Yet a further difference was that in this Example, the first distillation overhead stream containing 1,2-alkylene carbonate (and monopropylene glycol) was distilled in the second distillation column. In addition, in this Example, the final propylene carbonate product leaves the second distillation column as a bottoms stream, whereas in the Comparative Example, it leaves the second distillation column as an overhead stream. Comparison between the Example and Comparative Example

The Table below mentions some contaminants and the amounts thereof, which were found in samples taken from the final 1,2-alkylene carbonate products in the Example and the Comparative Example.

**Table**

| | Comparative Example | Example |
|---|---|---|
| acetaldehyde (mg/kg) | 6 | 2 |
| propylene oxide (mg/kg) | 1390 | 1 |
| propionaldehyde (mg/kg) | 2 | not detected |
| Allyl alcohol (mg/kg) | 6 | not detected |
| bromohydrin¹ (mg/kg, as bromine) | 18 | not detected |

| | | |
|---|---|---|
| ¹ Bromohydrin is a mixture of 1-bromo-2-hydroxy-propane and 2-bromo-1-hydroxy-propane. | | |

The above Table shows that with the present invention it is possible to substantially lower or even completely eliminate the amount of some contaminants in the final propylene carbonate product, more especially that of propylene oxide. Propylene oxide is presumably formed by a catalysed reverse reaction of propylene carbonate to propylene oxide and carbon dioxide near the reboilers of the distillation columns. Other contaminants, such as acetaldehyde, propionaldehyde and allyl alcohol, are presumably formed by thermal decomposition or catalysed reactions of propylene carbonate in the distillation columns.

Another contaminant is bromohydrin. It is presumably formed by degradation of the tetra(n-butyl)phosphonium bromide catalyst and subsequent reaction with propylene oxide. In the Comparative Example, all of this bromohydrin ends up in the final propylene carbonate product. In this way, the bromohydrin can hinder any subsequent process step, such as for example the reaction of the propylene carbonate product with an alkanol so as to obtain 1,2-propylene diol and a dialkylcarbonate. The bromohydrin may then also end up in the final dialkylcarbonate product. This is disadvantageous as for many applications wherein a dialkylcarbonate is used bromine free dialkylcarbonate is needed. On the other hand, in the final propylene carbonate product obtained in the Example, no bromohydrin was detected.

A further advantage is that in the Example bromohydrin is fully recycled from the second distillation column, via its overhead stream, to the carbonation reactor. It has been found that by recycling said halide by-product to the carbonation reactor together with the catalyst the catalytic behaviour of the system is improved. It is thought that bromohydrin is capable of re-activating any deactivated catalyst which presumably is in the form of HO-PBu₄, into active catalyst which presumably is in the form of Br-PBu₄.

## Claims

1. Process for preparing an 1,2-alkylene carbonate comprising
(i) contacting carbon dioxide, an 1,2-alkylene oxide and a carbonation catalyst in a reactor to produce a crude reactor effluent containing carbon dioxide, light components, 1,2-alkylene carbonate and catalyst;
(ii) separating carbon dioxide and light components from the crude reactor effluent to form a bottoms stream containing 1,2-alkylene carbonate and catalyst;
(iii) distilling the bottoms stream formed in step (ii) to form a first distillation overhead stream containing 1,2-alkylene carbonate and a first distillation bottoms stream containing catalyst, and recycling at least part of the first distillation bottoms stream to the reactor; and
(iv) distilling the first distillation overhead stream to form a second distillation overhead stream and a second distillation bottoms stream containing 1,2-alkylene carbonate, and recycling at least part of the second distillation overhead stream to the reactor.

2. Process according to claim 1, wherein an 1,2-alkylene diol is used as a solvent for the carbonation catalyst.

3. Process according to claim 2, wherein the first and second distillation overhead streams contain 1,2-alkylene diol.

4. Process according to claim 3, wherein at least part of the first distillation bottoms stream and at least part of the second distillation overhead stream are mixed prior to recycling to step (i).

5. Process according to any one of the preceding claims, wherein at least part of the light components and/or carbon dioxide separated in step (ii) is recycled to the reactor.

6. Process according to any one of the preceding claims, wherein an 1,2-alkylene carbonate is used as a solvent for the carbonation catalyst.

7. Process according to claim 6, wherein the first distillation bottoms stream contains part of 1,2-alkylene carbonate.

8. Process according to any one of the preceding claims, wherein the carbonation catalyst comprises a tetra-alkylphosphonium bromide.

9. Process according to any one of the preceding claims, wherein the 1,2-alkylene oxide is ethylene oxide and/or propylene oxide.

10. Process according to claim 2, wherein the 1,2-alkylene diol is monoethylene glycol and/or monopropylene glycol.

11. Process according to any one of claims 2 to 10, wherein the 1,2-alkylene oxide is ethylene oxide and the 1,2-alkylene diol is monoethylene glycol.

12. Process according to any one of claims 2 to 10, wherein the 1,2-alkylene oxide is propylene oxide and the 1,2-alkylene diol is monopropylene glycol.

13. Process according to any one of the preceding claims comprising
(v) contacting at least part of the second distillation bottoms stream formed in step (iv) with an alkanol to obtain a reaction mixture containing an 1,2-alkylene diol and a dialkylcarbonate; and
(vi) recovering 1,2-alkylene diol and dialkylcarbonate.

14. Process according to claim 13, wherein step (v) is performed in the presence of a heterogeneous transesterification catalyst.

15. Process according to claim 13 or 14, wherein the alkanol is methanol, ethanol or isopropanol.

## Patentansprüche

1. Verfahren zur Herstellung eines 1,2-Alkylencarbonats, umfassend
(i) Inkontaktbringen von Kohlendioxid, einem 1,2-Alkylenoxid und einem Karbonisierungskatalysator in einem Reaktor zur Erzeugung eines rohen Reaktorabstroms, der Kohlendioxid, leichte Komponenten, 1,2-Alkylencarbonat und Katalysator enthält;
(ii) Abtrennen von Kohlendioxid und leichten Komponenten aus dem rohen Reaktorabstrom zur Bildung eines Bodenstroms, der 1,2-Alkylencarbonat und Katalysator enthält;
(iii) Destillieren des im Schritt (ii) gebildeten Bodenstroms zur Bildung eines ersten Destillationskopfstroms, der 1,2-Alkylencarbonat enthält, und eines ersten Destillationsbodenstroms, der Katalysator enthält, und Rückführen mindestens eines Teils des ersten Destillationsbodenstroms zum Reaktor; und
(iv) Destillieren des ersten Destillationskopfstroms zur Bildung eines zweiten Destillationskopfstroms und eines zweiten Destillationsbodenstroms, der 1,2-Alkylencarbonat enthält, und Rückführen mindestens eines Teils des zweiten Destillationskopfstroms zum Reaktor.

2. Verfahren nach Anspruch 1, wobei ein 1,2-Alkylendiol als Lösungsmittel für den Carbonierungskatalysator verwendet wird.

3. Verfahren nach Anspruch 2, wobei der erste und zweite Destillationskopfstrom 1,2-Alkylendiol enthalten.

4. Verfahren nach Anspruch 3, wobei mindestens ein Teil des ersten Destillationsbodenstroms und mindestens ein Teil des zweiten Destillationskopfstroms vor dem Rückführen zu Schritt (i) gemischt werden.

5. Verfahren nach einem der vorhergehenden Ansprüche, wobei mindestens ein Teil der leichten Komponenten und/oder des Kohlendioxids, die in Stufe (ii) abgetrennt wurden, zum Reaktor zurückgeführt wird.

6. Verfahren nach einem der vorhergehenden Ansprüche, wobei ein 1,2-Alkylencarbonat als Lösungsmittel für den Carbonisierungskatalysator verwendet wird.

7. Verfahren nach Anspruch 6, wobei der erste Destillationbodenstrom einen Teil des 1,2-Alkylencarbonats enthält.

8. Verfahren nach einem der vorhergehenden Ansprüche, wobei der Carbonisierungskatalysator ein tetra-Alkylphosphoniumbromid umfasst.

9. Verfahren nach einem der vorhergehenden Ansprüche, wobei das 1,2-Alkylenoxid Ethylenoxid und/oder Propylenoxid ist.

10. Verfahren nach Anspruch 2, wobei das 1,2-Alkylendiol Monoethylenglycol und/oder Monopropylenglycol ist.

11. Verfahren nach einem der Ansprüche 2 bis 10, wobei das 1,2-Alkylenoxid Ethylenoxid ist und das 1,2-Alkylendiol Monoethylenglycol ist.

12. Verfahren nach einem der Ansprüche 2 bis 10, wobei das 1,2-Alkylenoxid Propylenoxid ist und das 1,2-Alkylendiol Monopropylenglycol ist.

13. Verfahren nach einem der vorhergehenden Ansprüche, umfassend
(v) Inkontaktbringen mindestens eines Teils des zweiten in Stufe (iv) gebildeten Destillationsbodenstroms mit einem Alkanol, um ein Reaktionsgemisch zu erhalten, das ein 1,2-Alkylendiol und ein Dialkylcarbonat enthält; und
(vi) Wiedergewinnen von 1,2-Alkylendiol und Dialkylcarbonat.

14. Verfahren nach Anspruch 13, wobei Schritt (v) in Gegenwart eines heterogenen Transveresterungskatalysators durchgeführt wird.

15. Verfahren nach Anspruch 13 oder 14, wobei das Alkanol Methanol, Ethanol oder Isopropanol ist.

## Revendications

1. Procédé de préparation d'un carbonate de 1,2-alkylène comprenant
(i) la mise en contact de dioxyde de carbone, d'un oxyde de 1,2-alkylène et d'un catalyseur de carbonatation dans un réacteur pour produire un effluant de réacteur brut contenant du dioxyde de carbone, des composants légers, du carbonate de 1,2-alkylène et un catalyseur ;
(ii) la séparation de dioxyde de carbone et des composants légers de l'effluant de réacteur brut pour former un courant inférieur contenant du carbonate de 1,2-alkylène et un catalyseur ;
(iii) la distillation du courant inférieur formé à l'étape (ii) pour former un premier courant supérieur de distillation contenant du carbonate de 1,2-alkylène et un premier courant inférieur de distillation contenant un catalyseur et le recyclage d'au moins une partie du premier courant inférieur de distillation au réacteur ; et
(iv) la distillation du premier courant supérieur de distillation pour former un deuxième courant supérieur de distillation et un deuxième courant inférieur de distillation contenant du carbonate de 1,2-alkylène et le recyclage d'au moins une partie du deuxième courant supérieur de distillation au réacteur.

2. Procédé selon la revendication 1, dans lequel un 1,2-alkylène-diol est utilisé comme solvant pour le catalyseur de carbonatation.

3. Procédé selon la revendication 2, dans lequel les premier et deuxième courants supérieurs de distillation contiennent un 1,2-alkylène-diol.

4. Procédé selon la revendication 3, dans lequel au moins une partie du premier courant inférieur de distillation et au moins une partie du deuxième courant supérieur de distillation sont mélangées avant le recyclage à l'étape (i).

5. Procédé selon l'une quelconque des revendications précédentes, dans lequel au moins une partie des composants légers et/ou du dioxyde de carbone séparé(s) à l'étape (ii) est recyclée dans le réacteur.

6. Procédé selon l'une quelconque des revendications précédentes, dans lequel un carbonate de 1,2-alkylène est utilisé comme solvant pour le catalyseur de carbonatation.

7. Procédé selon la revendication 6, dans lequel le premier courant inférieur de distillation contient une partie de carbonate de 1,2-alkylène.

8. Procédé selon l'une quelconque des revendications précédentes, dans lequel le catalyseur de carbonatation comprend un bromure de tétraalkylphosphonium.

9. Procédé selon l'une quelconque des revendications précédentes, dans lequel l'oxyde de 1,2-alkylène est l'oxyde d'éthylène et/ou l'oxyde de propylène.

10. Procédé selon la revendication 2, dans lequel le 1,2-alkylène-diol est le mono-éthylène glycol et/ou le monopropylène glycol.

11. Procédé selon l'une quelconque des revendications 2 à 10, dans lequel l'oxyde de 1,2-alkylène est l'oxyde d'éthylène et le 1,2-alkylène-diol est le mono-éthylène glycol.

12. Procédé selon l'une quelconque des revendications 2 à 10, dans lequel l'oxyde de 1,2-alkylène est l'oxyde de propylène et le 1,2-alkylène-diol est le monopropylène glycol.

13. Procédé selon l'une quelconque des revendications précédentes, comprenant
(v) la mise en contact d'au moins une partie du deuxième courant inférieur de distillation formé à l'étape (iv) avec un alcanol pour obtenir un mélange réactionnel contenant un 1,2-alkylène-diol et un dialkylcarbonate ; et
(vi) la récupération du 1,2-alkylène-diol et du dialkylcarbonate.

14. Procédé selon la revendication 13, dans lequel l'étape (v) est réalisée en présence d'un catalyseur de transestérification hétérogène.

15. Procédé selon la revendication 13 ou 14, dans lequel l'alcanol est le méthanol, l'éthanol ou l'isopropanol.
